# EUROPÄISCHE PATENTSCHRIFT

(11) **EP 0 593 590 B1**
(45) Veröffentlichungstag und Bekanntmachung des Hinweises auf die Patenterteilung: **29.11.1995**
(21) Anmeldenummer: 92914591.0
(22) Anmeldetag: 09.07.1992
(51) Int. Cl.: A61M 1/28, A61K 31/72

(54) **PHARMAZEUTISCHE ZUSAMMENSETZUNG FÜR DIE PERITONEALDIALYSE**
PHARMACEUTICAL PREPARATION FOR PERITONEAL DIALYSIS
COMPOSITION PHARMACEUTIQUE POUR LA DIALYSE PERITONEALE

(30) Priorität: 11.07.1991 DE 4123001
(43) Veröffentlichungstag der Anmeldung: 27.04.1994
(73) Patentinhaber: Laevosan-Gesellschaft m.b.H., A-4020 Linz (AT)
(72) Erfinder: FÖRSTER, Harald, D-6000 Frankfurt/Main 71 (DE); ASSKALI, Fatima, D-6000 Frankfurt/Main 71 (DE); NITSCH, Ernst, A-4040 Linz (AT)
(74) Vertreter: Huber, Bernhard, Dipl.-Chem.
(86) Internationale Anmeldenummer: EP9201551
(87) Internationale Veröffentlichungsnummer: WO9300939

(56) Entgegenhaltungen:
- EP-A- 0 170 275
- GB-A- 1 476 057

## Beschreibung

Die Erfindung betrifft pharmazeutische Zusammensetzungen, die ein Hydrokolloid enthalten, und ihre Verwendung für die Peritonealdialyse.

Bei der Peritonealdialyse (PD) handelt es sich um ein Verfahren zur Blutwäsche bei temporärer oder bei dauernder Niereninsuffizienz. Die Funktion der Nieren besteht darin, Stoffwechselendprodukte (Harnstoff oder Harnsäure) oder mit der Nahrung zugeführte Substanzen (z.B. Kalium) aus dem Körper zu entfernen. Bei Ausfall der Nieren kommt es zur Vergiftung des Organismus durch Anreicherung solcher Substanzen, sofern keine Ersatzmaßnahmen ergriffen werden. Als Ersatzmaßnahme für den teilweisen oder vollständigen Ausfall der Nieren kommen neben der Peritonealdialyse noch die Hämofiltration und die Hämodialyse (Blutwäsche) in Frage. Diese Alternativmöglichkeiten zur Peritonealdialyse sind mit größerem apparativen Aufwand und mit der Verfügbarkeit eines Blutgefäßzuganges verbunden, was Nachteile für den Patienten bedeutet.

Demgegenüber hat die Peritonealdialyse, insbesondere in Form der kontinuierlichen ambulanten Peritonealdialyse (CAPD, continuous ambulatory peritoneal dialysis) den Vorteil der geringeren Beeinträchtigung der Patienten und der fehlenden Abhängigkeit von stationären Apparaten. Der Nachteil der konventionellen hyperosmolaren Peritonealdialyse ist die Schädigung des Peritonealepithels durch die Verwendung von hyperosmolaren Lösungen, mit denen eine Ausscheidung der gewünschten Substanzen aus dem Blut in die Peritonealhöhle erreicht wird. Bei der konventionellen Peritonealdialyse ist es erforderlich, diesen Effekt durch den Zuatz von 1 bis 5 Gew.-% Glucose oder andere osmotisch wirksame Substanzen (z.B. Sorbit) zur Spüllösung zu erzielen. Durch den Übertritt von Glucose aus der Bauchhöhle in den Organismus wird ein nutritiver Effekt bewirkt, der unter Umständen erhebliche Bedeutung haben kann und unerwünscht ist.

Aufgabe der vorliegenden Erfindung ist es deshalb, pharmazeutische Zusammensetzungen für die Peritonealdialyse bereitzustellen, die die vorstehend genannten Nachteile der bisher üblichen PD und insbesondere der kontinuierlichen ambulanten Peritonealdialyse vermeiden. Diese Aufgabe wird mit der vorliegenden Erfindung gelöst.

Gegenstand der Erfindung gemäß Patentanspruch 1 ist eine pharmazeutische Zusammensetzung zur Peritonealdialyse, enthaltend einen Stärkeester als kolloid-osmotisch wirksame Substanz, bei welchem die Stärke mit Acylgruppen von Monocarbonsäuren odcr Dicarbonsäuren oder Gemischen aus Mono- und Dicarbonsäuren mit jeweils 2 bis 6 C-Atomen substituiert ist, in einer Menge von 1 bis 12 Gew.-% in Kombination mit einem physiologisch annehmbaren Elektrolyten und/oder einer oder mehreren anderen osmotisch wirksamen Substanzen.

Zweckmäßige Ausgestaltungen davon sind Gegenstand der Ansprüche 2 bis 7.

Es ist bekannt, Stärkederivate, Dextrane und Gelatine, vorzugsweise mit einem Molekulargewicht ≧ 40000, als Blutplasmaersatz (Plasmastreckmittel) zu verwenden (vgl. z.B. US-A 3,937,821; DE-A 33 13 600; Römpp Chemielexikon, 9. Auflage, Seite 919, 1509).

Es wurde nun gefunden, daß sich Stärkeester, wie sie in Anspruch 1 definiert sind und die sich durch eine große Wasserbindungsfähigkeit auszeichnen, sehr gut zur Verwendung in der Peritonealdialyse eignen.

Erfindungsgemäß ist es möglich, durch Verzicht auf hyperosmolare Lösungen oder, bei einer Kombination mit konventionell verwendeten osmotisch wirksamen Substanzen, durch eine wesentliche Einschränkung der Hyperosmolarität der Spüllösungen, insbesondere durch einen Verzicht auf Glucose oder eine Verringerung der Konzentration von Glucose als bisher wesentlichen Bestandteil, eine sanftere dialysierende Wirkung ohne Schädigung des Peritonealepithels und mit geringerer nutritiver Wirkung zu erreichen. Als wirksame Kraft für die Ausscheidung von Stoffwechselendprodukten in die Peritonealhöhle ist erfindungsgemäß nicht die osmotische Druckdifferenz oder die kolloidosmotische Druckdifferenz von Bedeutung, sondern die Wasserbindungsfähigkeit der Acylstärke in der Peritonealhöhle. Der Hydrokolloideffekt bewirkt über eine Wasserbindung in der Peritonealhöhle einen sogenannten "solvent drag", der mit der Wirkung einer osmotischen Druckdifferenz vergleichbar ist. Dieser "solvent drag" führt zu einer Ausscheidung von Substanzen in die Peritonealhöhle und übt damit die dialysierende Wirkung aus.

Gegenstand der Erfindung ist deshalb auch die Verwendung der vorstehend beschriebenen erfindungsgemäßen pharmazeutischen Zusammensetzung für die Peritonealdialyse.

Das Molekulargewicht (Gewichtsmittel, M̅w̅) beträgt vorzugsweise > 1000 Dalton. Die Obergrenze des Molekulargewichtsbereiches der Stärkeester ist dabei unkritisch und insbesondere davon abhängig, daß sie nicht zu Ablagerungen im Organismus führen sollen. In der Regel liegt die Molekulargewichts-Obergrenze, die auch von der Art des Stärkeesters abhängt, bei ca. 1000000 Dalton. Vorzugsweise wird ein Molekulargewicht (M̅w) von ca. 100000 bis 200000 Dalton verwendet.

Die erfindungsgemäß besonders bevorzugten Stärkeester sind Stärkeester mit einer molaren Substitution von 0,1 bis 1,5. Stärkeester sind Ester mit organischen Carbonsäuren und insbesondere mit aliphatischen Mono- und Dicarbonsäuren mit 2 bis 6 Kohlenstoffatomen, wie z.B. Essigsäure, Propionsäure, Buttersäure, Isobuttersäure und insbesondere Essigsäure. Die molare Substitution MS beträgt vorzugsweise 0,3 bis 0,5. Ein erfindungsgemäß besonders bevorzugter Stärkeester ist Acetylstärke, insbesondere mit einem Molekulargewicht (M̅w) von 100000 bis 200000 Dalton und einer Substitution MS von 0,3 bis 0,5.

Die erfindungsgemäße pharmazeutische Zusammensetzung zur Peritonealdialyse enthält weiter einen physiologisch annehmbaren Elektrolyten und/oder eine andere osmotisch wirksame Substanz. Die Konzentration an Stärkeester ist vorzugsweise 1 bis 12 % Gew/Vol, insbesondere 2 bis 6 % Gew/Vol, bezogen auf die gesamte pharmazeutische Zusammensetzung.

Als physiologisch annehmbare Elektrolyte kommen solche Elektrolyte und deren Gemische in Frage, wie sie üblicherweise in Zusammensetzungen zur Peritonealdialyse verwendet werden, also insbesondere z.B. Natriumchlorid, Calciumchlorid, Salze der niederen Carbonsäuren mit 2 bis 4 Kohlenstoffatomen, insbesondere Essigsäure.

Die andere osmotisch wirksame Substanz kann eine niedermolekulare organische Verbindung sein, insbesondere eine solche, wie sie z.B. bei der konventionellen hyperosmolaren Peritonealdialyse verwendet wird und besteht vorzugsweise aus mehrwertigen Alkoholen, Monosacchariden, Disacchariden, wie z.B. Glycerin, Sorbit, Maltose und in erster Linie Glucose und/oder Aminosäuren.

Die erfindungsgemäßen pharmazeutischen Zusammensetzungen können ein oder mehrere erfindungsgemäße Stärkeester in Kombination mit einer oder mehreren der osmotisch wirksamen Substanzen in Wasser enthalten.

Die erfindungsgemäß eingesetzten Stärkeester können nach dem in der gleichzeitig unter der Bezeichnung "Verfahren zur Herstellung von Stärkeestern für klinische, insbesondere parenterale Anwendung" der gleichen Anmelderin, Aktenzeichen P 4123000.0 hergestellt werden. Die Herstellung der pharmazeutischen Zusammensetzung erfolgt auf an sich bekannte Weise, z.B. durch Mischen der Komponenten und des pharmazeutischen Trägers, wobei die Stärkeester vorzugsweise in Form von durch Trocknen, z.B. durch Sprühtrocknung, Trommeltrocknung oder Vakuumtrocknung und Mahlen erhaltene Pulver eingesetzt werden.

Ein weiterer Gegenstand der Erfindung ist daher die Verwendung eines Stärkeesters, bei dem die Stärke mit Acylgruppen von Monocarbonsäuren oder Dicarbonsäure oder ein Gemisch von Mono- und Dicarbonsäuren mit jeweils 2 bis 6 C-Atomen substituiert ist, zur Herstellung einer pharmazeutischen Zusammensetzung zur Peritonealdialyse gemäß Anspruch 9. Zweckmäßige Ausgestaltungen dieser Verwendung sind in den Unteransprüchen 10 bis 15 aufgeführt.

Während der Dialyse erfolgt ein Übertritt des in der Spüllösung verwendeten Hydrokolloids aus der Peritonealhöhle in das Blut. Deshalb sind besonders solche Stärkeester geeignet, die im Organismus abgebaut und damit auch bei lang dauernder Behandlung nicht gespeichert werden.

Ein erfindungsgemäß besonders bevorzugter Stärkeester ist Acetylstärke. Diese Stärkeester können durch den Einsatz von körpereigenen Enzymen abgebaut und metabolisiert werden. Aufgrund dieses physiologischen Abbaus wird neben Oligosacchariden, Isomaltose und Maltose auch Glucose gebildet. Deshalb kann eine Peritonealdialyse auch ohne Zusatz von Glucose (glucosefreie Peritonealdialyse) durchgeführt werden. Ein Teil der bei dem Abbau des Stärkeesters, z.B. Acetylstärke, gebildeten Glucose wird noch während der Dialyse in die Peritonealhöhle ausgeschieden, wodurch der nutritive Effekt der Peritonealdialyse mit Stärkeester eingeschränkt werden kann. Eine Einwirkung der mit Stärkeester durchgeführten Peritonealdialyse auf den Fettstoffwechsel von Versuchstieren wurde nicht festgestellt. Die "glucosefreie Peritonealdialyse" besitzt deshalb deutlich Vorteile im Vergleich zur konventionellen Peritonealdialyse, bei der höherprozentige Glucoselösungen eingesetzt werden. Durch den Abbau mit körpereigenen Enzymen wird außerdem eine Speicherung von Stärkeestern verhindert. Auch nach 5-tägiger Peritonealdialyse mit Acetylstärke wurde - zum Unterschied von Hydroxyethylstärke - in den Organen der untersuchten Versuchstiere kein Polysaccharid in höherer Konzentration festgestellt.

Die vorstehend angegebenen Ergebnisse beruhen auf tierexperimentellen Untersuchungen an beidseits nephrektomierten Ratten. Bei den Tieren wurde durch Verwendung einer 3 %igen Acetylstärkelösung mit Zusatz entsprechender Elektrolyte eine Reinigung des Blutes von Harnstoff als Referenzsubstanz oder von entsprechenden Elektrolyten (z.B. Kalium) erreicht. Durch tägliche Peritonealdialyse konnte bei den Tieren über einen Zeitraum von 5 Tagen ein ausreichender Gesundheitszustand aufrecht erhalten werden. Vergleichsuntersuchungen wurden mit Lösungen durchgeführt, die Glucose (2 % Gew./Vol.) enthielten. Außerdem wurden Untersuchungen mit Lösungen vorgenommen, die eine Kombination an erfindungsgemäßer Acetylstärke mit 1 Gew.-% Glucose als anderer osmotisch wirksamer Substanz enthielten. Es wurden in allen Fällen Dialyseeffekte beobachtet, die nicht nur auf Unterschiede im osmotischen oder im kolloidosmotischen Druck zurückzuführen waren. Auch bei der Verwendung von isomolaren erfindungsgemäßen Hydrokolloidlösungen (d.h. ohne osmotisch wirksamen Gradienten) wurde eine Angleichung der Plasmakonzentrationen und der Spüllösungskonzentrationen erreicht. Damit wird bewiesen, daß der Hydrokolloideffekt eine Wasserbindung in der Peritonealhöhle bewirkt und über den sogenannten "solvent drag" die einer osmotischen Druckdifferenz entsprechende dialysierende Wirkung ausübt.

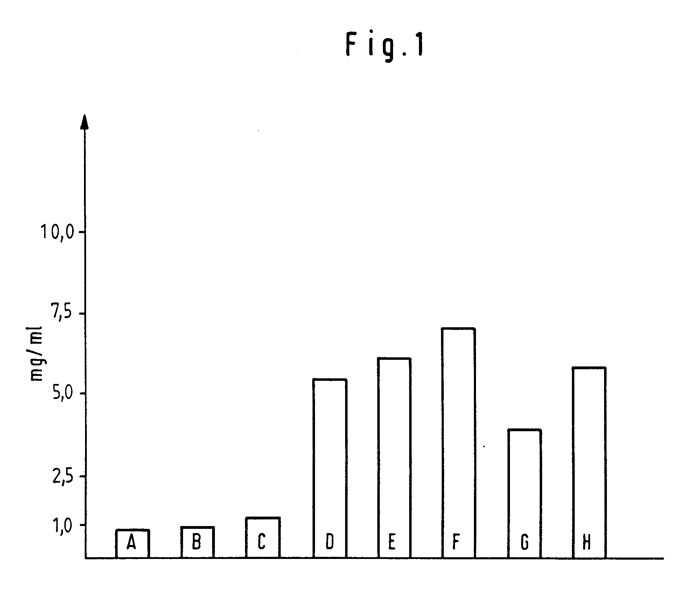

Auch nach einer 5-tägigen erfolgreichen Peritonealdialyse mit Acetylstärkelösungen wurden nur Spuren von Acetylstärke im Serum der dialysierten Tiere festgestellt (vgl. Fig. 1). Bei der Untersuchung der Organe wurde weder in der Milz noch in der Leber oder in den Lungen Acetylstärke nachgewiesen. Aus diesen Untersuchungen ist deshalb zu schließen, daß Acetylstärke, die während der Peritonealdialyse in den Blutkreislauf gelangt, rasch und vollständig durch körpereigene Enzyme abgebaut wird.

Die nachfolgenden Beispiele sollen die Erfindung näher erläutern.

### Beispiel 1

### Herstellung von Acetylstärke (AST)-Lösungen für die kontinuierliche ambulante Peritonealdialyse (CAPD)

Es wurden je 250 ml der CAPD-Lösungen gemäß der nachstehenden Tabelle 2 hergestellt. Dabei wurde die in Tabelle 1 angegebene Elektrolytmischung verwendet. Als Acetylstärke (AST) wurde Acetylamylopektin mit einer molaren Substitution MS von 0,355 und einem Mw von ca. 200000 Dalton eingesetzt. Die Lösungen wurden in 20 ml-Durchstichfläschchen abgefüllt.

Aus Tabelle 2 geht hervor, daß die so hergestellten Lösungen eine gute Stabilität aufweisen. Auch nach Sterilisation war nur eine geringgradige Verseifung der Estergruppen feststellbar.

**Tabelle 1**

| (Elektrolytmischung für CAPD-Lösungen mit AST) | | |
|---|---|---|
| M | mMol/l | g/l |
| 58,443 Natriumchlorid | 120 | 7,013 |
| 136,080 Na-acetat.3H₂O | 25 | 3,402 |
| 147,020 Ca-chlorid.2H₂O | 2 | 0,294 |
| 60,053 Essigsäure | 5 | 0,303 |
| Osmolarität mOsmol/l | 301 | |

**Tabelle 2**

| (CAPD-Lösungen mit AST) | | | | | | | | | | |
|---|---|---|---|---|---|---|---|---|---|---|
| Nr. | AST g/l | Gluc g/l | mOsmol/l | pH | Essigsäure | | Restacetylg. Mol/Mol | M̅w.10⁻³* | M̅n.10⁻³ | |
| | | | | | freie mMol/l | verest. mMol/l | | | | |
| 1/1 | 30 | - | 301 | 5,49 | 3,28 | 53,66 | - | 313 | 153 | v.Steril. |
| | | | | 5,25 | 5,34 | 51,51 | 0,96 | 230 | 140 | 30′100° |
| | | | | 4,85 | 11,49 | 45,17 | 0,84 | 219 | 134 | 8′121° |
| 1/2 | 30 | 0,901 | 306 | 5,44 | 3,28 | 53,88 | - | 306 | 155 | v.Steril. |
| | | | | 5,21 | 5,44 | 51,51 | 0,96 | 233 | 142 | 30′100° |
| | | | | 4,86 | 11,49 | 46,24 | 0,86 | 216 | 134 | 8′121° |
| 2/1 | 45 | - | 301 | 5,46 | 3,39 | 78,61 | - | 325 | 153 | v.Steril. |
| | | | | 5,19 | 5,95 | 76,68 | 0,98 | 203 | 130 | 30′100° |
| | | | | 4,79 | 14,06 | 70,01 | 0,89 | 223 | 136 | 8′121° |
| 2/2 | 45 | 0,901 | 306 | 5,48 | 3,39 | 78,29 | - | 275 | 147 | v.Steril. |
| | | | | 5,18 | 6,36 | 76,14 | 0,97 | 218 | 136 | 30′100° |
| | | | | 4,81 | 14,06 | 68,72 | 0,88 | 226 | 138 | 8′121° |

| | | | | | | | | | | |
|---|---|---|---|---|---|---|---|---|---|---|
| * Die höheren M̅w's u. M̅n's vor Sterilisation dürften vorwiegend durch Aggregatbildung bedingt sein. | | | | | | | | | | |

Alle Lösungen waren auch nach der Sterilisation nahezu farblos. Durch die Sterilisation trat, wie zu erwarten, eine geringfügige Acetylabspaltung auf. Der Restacetylgehalt betrug bei schonender Sterilisation (30 Min. bei 100°C) ca. 96 %, nach einer Sterilisation während 8 Min. bei 121°C ca. 86 %. Eine schonende Sterilisation ist deshalb zweckmäßig.

Auf ähnliche Weise wurden folgende weitere erfindungsgemäße Lösungen zur Perltonealdialyse unter Verwendung der in Tabelle 1 angegebenen Elektrolyten hergestellt:
1. 3 %ige Acetylstärke-Lösung (molare Substitution 0,3 oder 0,5)
2. Kombinationslösung mit 1,5 % Acetylstärke und 1 % Glucose (als Beispiel einer osmotisch wirksamen Substanz)
3. 3 %ige Hydroxyethylstärke-Lösungen HES mit 200/0,5 (Gewichtsmittel des Molekulargewichts ca. 200000 Dalton, molare Substitution 0,5 Mol), HES 100/0,7, HES 40/0,5, HES 70/0,7 und HES 450/0,7.
4. 3 %ige Hydroxyethylstärke-Lösungen (HES 40.0.5 und HES 200.05) mit 1 % Glucose.

### Beispiel 2

### Durchführung der Peritonealdialyse

Wistar-Ratten mit einem Körpergewicht von 250 bis 350 g werden in Narkose beidseits nephrektomiert. Ferner werden in die Bauchhöhle zwei Verweilkatheter mit Ableitung nach außen operativ eingebracht. Als Ersatz der Nierenfunktion werden täglich 6 bis 8 Spülungen der Bauchhöhle durch Einbringen von jeweils 60 bis 100 ml Spüllösung vorgenommen. Nach einem Zeitraum von jeweils 30 bis 60 Min. wird die Spüllösung abgelassen.

Mit dieser Behandlung können die Tiere trotz fehlender Nieren über längere Zeiträume am Leben und bei Gesundheit gehalten werden. Als Versuchszeitraum wurden 5 Dialysetage gewählt. Am 5. Tag wurden unmittelbar vor Dialysebeginn Blutentnahmen aus der Orbita durchgeführt. Nach der letzten Dialyse des Tages wurden die Tiere in Narkose durch Ausbluten aus der Bauchaorta getötet. Anschließend wurden die folgenden Organe für weitere Untersuchungen entnommen: Milz, Leber, Lungen.

Die Figur 1 zeigt den Serumgehalt (in mg/ml) nach der 5. Dialyse (5. Tag).

Die Figur 2 zeigt den Gehalt an Hydrokolloid in der Milz (in mg/g) nach der 5. Dialyse (5. Tag).

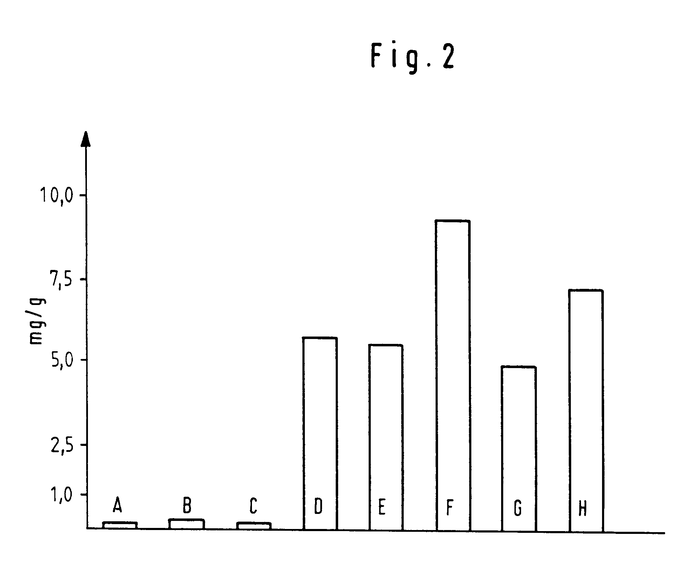

In den Fig. 1 und 2 bedeuten:
A, B, C: 3 % Acetylstärke (Acetylamylopektin)-Lösungen ohne Glucosezusatz,
D bis H: 3 %ige Hydroxyethylstärke-Lösungen ohne Glucosezusatz und zwar mit HES 100/0,7 (D), HES 40/0,5 (E), HES 70/0,7 (F), HES 200/0,5 (G) und HES 450/0,7 (H).

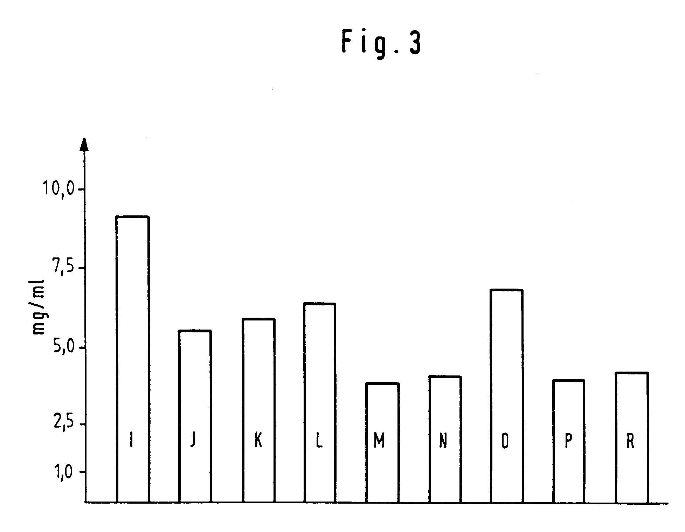

Die Fig. 3 zeigt den Harnstoffgehalt im Serum vor und nach der 5. Dialyse (5. Tag) und im Dialysat.

Es bedeuten:
I, J, K: 3 % Acetylstärke-Lösung ohne Glucose (Extraktion 36 %), und zwar den Serumgehalt vor der Dialyse (I), nach der Dialyse (J) und im Dialysat (K);
L, M, N: 3 % HES 40/0,5-Lösung mit 1 % Glucose (Extraktion 39,4 %), und zwar den Serumgehalt vor der Dialyse (L), nach der Dialyse (M) und im Dialysat (N);
O, P, R: 3 % HES 200/0,5-Lösung mit 1 % Glucose (Extraktion 42 %), und zwar den Serumgehalt vor der Dialyse (O), nach der Dialyse (P) und im Dialysat (R).

## Patentansprüche (Patentansprüche für folgende(n) Vertragsstaat(en): AT, BE, CH, LI, DE, DK, FR, GB, IT, LU, MC, NL, SE)

1. Pharmazeutische Zusammensetzung für die Peritonealdialyse, enthaltend einen Stärkeester als kolloid-osmotisch wirksame Substanz, bei welchem die Stärke mit Acylgruppen von Monocarbonsäuren oder Dicarbonsäuren oder Gemischen aus Mono- und Dicarbonsäuren mit jeweils 2 bis 6 C-Atomen substituiert ist, in einer Menge von 1 bis 12 Gew.-% in Kombination mit einem physiologisch annehmbaren Elektrolyten und/oder einer oder mehreren anderen osmotisch wirksamen Substanzen.

2. Pharmazeutische Zusammensetzung nach Anspruch 1,
**dadurch gekennzeichnet,**
daß der Stärkeester ein Molekulargewicht (M̅w̅) von mehr als 1000 Dalton, insbesondere 100000 bis 200000 Dalton und eine molare Substitution von 0,1 bis 1,5, insbesondere von 0,3 bis 0,5 aufweist.

3. Pharmazeutische Zusammensetzung nach Anspruch 1 oder 2,
**dadurch gekennzeichnet,**
daß der Stärkeester Acetylstärke ist.

4. Pharmazeutische Zusammensetzung nach Anspruch 2 oder 3,
**dadurch gekennzeichnet,**
daß die Acetylstärke eine Acetylstärke mit einem Molekulargewicht (M̅w̅) von 100000 bis 200000 Dalton und einer molaren Substitution von 0,1 bis 0,7, insbesondere von 0,3 bis 0,5 ist.

5. Pharmazeutische Zusammensetzung nach einem der Ansprüche 1 bis 4,
**dadurch gekennzeichnet,**
daß sie 2 bis 6 Gew.-% des Stärkeesters enthält.

6. Pharmazeutische Zusammensetzung nach Anspruch 5,
**dadurch gekennzeichnet,**
daß die anderen osmotisch wirksamen Substanzen mehrwertige Alkohole, Monosaccharide, Disaccharide und/oder Aminosäuren sind.

7. Pharmazeutische Zusammensetzung nach Anspruch 6,
**dadurch gekennzeichnet,**
daß die andere osmotisch wirksame Substanz Glucose ist.

8. Verwendung einer in einem der Ansprüche 1 bis 7 definierten pharmazeutischen Zusammensetzung für die Peritonealdialyse.

9. Verwendung eines Stärkeesters, bei dem die Stärke mit Acylgruppen von Monocarbonsäuren oder einem Gemisch aus Mono- und Dicarbonsäuren mit jeweils 2 bis 6 C-Atomen substituiert ist, zur Herstellung einer pharmazeutischen Zusammensetzung für die Peritonealdialyse.

10. Verwendung nach Anspruch 9,
**dadurch gekennzeichnet,**
daß man einen Stärkeester verwendet, der ein Molekulargewicht von mehr als 1000 Dalton, insbesondere 100000 bis 200000 Dalton und eine molare Substitution (Ms) von 0,1 bis 1,5, insbesondere 0,3 bis 0,5 aufweist.

11. Verwendung nach Anspruch 10,
**dadurch gekennzeichnet,**
daß der Stärkeester Acetylstärke ist.

12. Verwendung nach Anspruch 11,
**dadurch gekennzeichnet,**
daß die Acetylstärke ein Molekulargewicht von 100000 bis 200000 Dalton und eine molare Substitution von 0,1 bis 0,7, insbesondere 0,3 bis 0,5 hat.

13. Verwendung nach einem der Ansprüche 10 bis 12,
**dadurch gekennzeichnet,**
daß man einen physiologisch annehmbaren Elektrolyten und/oder eine andere osmotisch wirksame Substanz zusetzt.

14. Verwendung nach Anspruch 13,
**dadurch gekennzeichnet,**
daß man als andere osmotisch wirksame Substanz einen mehrwertigen Alkohol, ein Monosaccharid, Disaccharid und/oder eine Aminosäure zusetzt.

15. Verwendung nach Anspruch 14,
**dadurch gekennzeichnet,**
daß man Glucose zusetzt.

## Patentansprüche (Patentansprüche für folgende(n) Vertragsstaat(en): GR, ES)

1. Verfahren zur Herstellung einer pharmazeutischen Zusammensetzung für die Peritonealdialyse,
**dadurch gekennzeichnet,**
daß man einen Stärkeester als kolloid-osmotisch wirksame Substanz, bei welchem die Stärke mit Acylgruppen von Monocarbonsäuren oder Dicarbonsäuren oder Gemischen aus Mono- und Dicarbonsäuren mit jeweils 2 bis 6 C-Atomen substitutiert ist, in einer Menge von 1 bis 12 Gew.-% mit einem physiologisch annehmbaren Elektrolyten und/oder einer oder mehreren anderen osmotisch wirksamen Substanzen kombiniert.

2. Verfahren nach Anspruch 1,
**dadurch gekennzeichnet,**
daß man einen Stärkeester verwendet, der ein Molekulargewicht (M̅w̅) von mehr als 1000 Dalton, insbesondere 100000 bis 200000 Dalton und eine molare Substitution von 0,1 bis 1,5, insbesondere von 0,3 bis 0,5 aufweist.

3. Verfahren nach Anspruch 1 oder 2,
**dadurch gekennzeichnet,**
daß man als Stärkeester Acetylstärke verwendet.

4. Verfahren nach Anspruch 2 oder 3,
**dadurch gekennzeichnet,**
daß man als Acetylstärke eine Acetylstärke mit einem Molekulargewicht (M̅w̅) von 100000 bis 200000 Dalton und einer molaren Substitution von 0,1 bis 0,7, insbesondere von 0,3 bis 0,5 verwendet.

5. Verfahren nach einem der Ansprüche 1 bis 4,
**dadurch gekennzeichnet,**
daß man 2 bis 6 Gew.-% des Stärkeesters verwendet.

6. Verfahren nach Anspruch 5,
**dadurch gekennzeichnet,**
daß man als weitere osmotisch wirksame Substanzen mehrwertige Alkohole, Monosaccharide, Disaccharide und/oder Aminosäuren verwendet.

7. Verfahren nach Anspruch 6,
**dadurch gekennzeichnet,**
daß man als andere osmotisch wirksame Substanz Glucose verwendet.

## Claims (Claims for the following Contracting State(s): AT, BE, CH, LI, DE, DK, FR, GB, IT, LU, MC, NL, SE)

1. Pharmaceutical preparation for peritoneal dialysis containing a starch ester as a colloid-osmotically active substance in which the starch is substituted with acyl groups or monocarboxylic acids or dicarboxylic acids or mixtures of mono- and dicarboxylic acids each with 2 to 6 C atoms in an amount of 1 to 12 % by weight in combination with a physiologically acceptable electrolyte and/or one or several other osmotically active substances.

2. Pharmaceutical composition as claimed in claim 1,
**wherein**
the starch ester has a molecular weight (M̅w̅) of more than 1000 Daltons, in particular 100000 to 200000 Daltons and a molar substitution of 0.1 to 1.5, in particular of 0.3 to 0.5.

3. Pharmaceutical composition as claimed in claim 1 or 2,
**wherein**
the starch ester is acetyl starch.

4. Pharmaceutical composition as claimed in claim 2 or 3,
**wherein**
the acetyl starch is an acetyl starch with a molecular weight (M̅w̅) of 100000 to 200000 Daltons and a molar substitution of 0.1 to 0.7, in particular of 0.3 to 0.5.

5. Pharmaceutical composition as claimed in one of the claims 1 to 4,
**wherein**
it contains 1 to 12 % by weight, in particular 2 to 6 % by weight of the starch ester in combination with a physiologically acceptable electrolyte and/or one or several other osmotically active substances for peritoneal dialysis.

6. Pharmaceutical composition as claimed in claim 5,
**wherein**
the other osmotically active substances are polyvalent alcohols, monosaccharides, disaccharides and/or amino acids.

7. Pharmaceutical composition as claimed in claim 6,
**wherein**
the other osmotically active substance is glucose.

8. Use of a pharmaceutical composition as defined in one of the claims 1 to 7 for peritoneal dialysis.

9. Use of a starch ester in which the starch is substituted with acyl groups of monocarboxylic acids or a mixture of mono- and dicarboxylic acids each with 2 to 6 C atoms for the production of a pharmaceutical composition for peritoneal dialysis.

10. Use as claimed in claim 9,
**wherein**
a starch ester is used which has a molecular weight of more than 1000 Daltons, in particular 100000 to 200000 Daltons and a molar substitution (Ms) of 0.1 to 1.5, in particular of 0.3 to 0.5.

11. Use as claimed in claim 10,
**wherein**
the starch ester is acetyl starch.

12. Use as claimed in claim 11,
**wherein**
the acetyl starch has a molecular weight of 100000 to 200000 Daltons and a molar substitution of 0.1 to 0.7, in particular 0.3 to 0.5.

13. Use as claimed in one of the claims 10 to 12,
**wherein**
a physiologically acceptable electrolyte and/or another osmotically active substance is added.

14. Use as claimed in claim 13,
**wherein**
a polyvalent alcohol, a monosaccharide, disaccharide and/or an amino acid is added as the other osmotically active substance.

15. Use as claimed in claim 14,
**wherein**
glucose is added.

## Claims (Claims for the following Contracting State(s): GR, ES)

1. Process for the production of a pharmaceutical preparation for peritoneal dialysis
**wherein**
a starch ester as a colloid-osmotically active substance in which the starch is substituted with acyl groups or monocarboxylic acids or dicarboxylic acids or mixtures of mono- and dicarboxylic acids each with 2 to 6 C atoms in an amount of 1 to 12 % by weight in combination with a physiologically acceptable electrolyte and/or one or several other osmotically active substances.

2. Process as claimed in claim 1,
**wherein**
a starch ester is used that has a molecular weight (M̅w̅) of more than 1000 Daltons, in particular 100000 to 200000 Daltons and a molar substitution of 0.1 to 1.5, in particular of 0.3 to 0.5.

3. Process as claimed in claim 1 or 2,
**wherein**
acetyl starch is used as the starch ester.

4. Process as claimed in claim 2 or 3,
**wherein**
an acetyl starch is used as the acetyl starch with a molecular weight (M̅w̅) of 100000 to 200000 Daltons and a molar substitution of 0.1 to 0.7, in particular of 0.3 to 0.5.

5. Process as claimed in one of the claims 1 to 4,
**wherein**
it contains 2 to 6 % by weight of the starch ester.

6. Process as claimed in claim 5,
**wherein**
polyvalent alcohols, monosaccharides, disaccharides and/or amino acids are used as the other osmotically active substances.

7. Process as claimed in claim 6,
**wherein**
glucose is used as the other osmotically active substance.

## Revendications (Revendications pour l'(les) Etat(s) contractant(s) suivant(s): AT, BE, CH, LI, DE, DK, FR, GB, IT, LU, MC, NL, SE)

1. Composition pharmaceutique pour la dialyse péritonéale, contenant comme substance à activité colloïde-osmotique un ester d'amidon dans lequel l'amidon est substitué par des groupes acyles d'acides monocarboxyliques ou d'acides dicarboxyliques ou de mélanges d'acides mono- et dicarboxyliques ayant chacun de 2 à 6 atomes de carbone, en une quantité de 1 à 12% en poids, en combinaison avec un électrolyte physiologiquement acceptable et/ou une ou plusieurs autres substances à activité osmotique.

2. Composition pharmaceutique selon la revendication 1, caractérisée en ce que l'ester d'amidon a une masse moléculaire (M̅w̅) de plus de 1000 daltons, en particulier de 100 000 à 200 000 daltons, et une substitution molaire de 0,1 à 1,5, en particulier de 0,3 à 0,5.

3. Composition pharmaceutique selon la revendication 1 ou 2, caractérisée en ce que l'ester d'amidon est un amidon acétylé.

4. Composition pharmaceutique selon la revendication 2 ou 3, caractérisée en ce que l'amidon acétylé est un amidon acétylé ayant une masse moléculaire (M̅w̅) de 100 000 à 200 000 daltons et une substitution molaire de 0,1 à 0,7, en particulier de 0,3 à 0,5.

5. Composition pharmaceutique selon l'une des revendications 1 à 4, caractérisée en ce qu'elle contient 2 à 6% en poids de l'ester d'amidon.

6. Composition pharmaceutique selon la revendication 5, caractérisée en ce que les autres substances à activité osmotique sont des polyols, des monosaccharides, des disaccharides et/ou des aminoacides.

7. Composition pharmaceutique selon la revendication 6, caractérisée en ce que l'autre substance à activité osmotique est le glucose.

8. Utilisation d'une composition pharmaceutique définie dans l'une des revendications 1 à 7 pour la dialyse péritonéale.

9. Utilisation d'un ester d'amidon dans lequel l'amidon est substitué par des groupes acyles d'acides monocarboxyliques ou d'acides dicarboxyliques ou de mélanges d'acides mono- et dicarboxyliques ayant chacun de 2 à 6 atomes de carbone, pour la préparation d'une composition pharmaceutique pour la dialyse péritonéale.

10. Utilisation selon la revendication 9, caracterisée en ce que l'on utilise un ester d'amidon qui a une masse moléculaire de plus de 1000 daltons, en particulier de 100 000 à 200 000 daltons, et une substitution molaire (Ms) de 0,1 à 1,5, en particulier de 0,3 à 0,5.

11. Utilisation selon la revendication 10, caractérisée en ce que l'ester d'amidon est un amidon acétylé.

12. Utilisation selon la revendication 11, caractérisée en ce que l'amidon acétylé' a une masse moléculaire de 100 000 à 200 000 daltons et une substitution molaire de 0,1 à 0,7, en particulier de 0,3 à 0,5.

13. Utilisation selon l'une des revendications 10 à 12, caractérisée en ce que l'on ajoute un électrolyte physiologiquement acceptable et/ou une autre substance à activité osmotique.

14. Utilisation selon la revendication 13, caractérisée en ce que l'on ajoute comme autre substance à activité osmotique un polyol, un monosaccharide, un disaccharide et/ou un aminoacide.

15. Utilisation selon la revendication 14, caractérisée en ce que l'on ajoute du glucose.

## Revendications (Revendications pour l'(les) Etat(s) contractant(s) suivant(s): GR, ES)

1. Procédé de préparation d'une composition pharmaceutique pour la dialyse péritonéale, caractérisé en ce que l'on combine, comme substance à activité colloïde-osmotique, un ester d'amidon dans lequel l'amidon est substitué par des groupes acyles d'acides monocarboxyliques ou d'acides dicarboxyliques ou de mélanges d'acides mono- et dicarboxyliques ayant chacun de 2 à 6 atomes de carbone, en une quantité de 1 à 12% en poids, en combinaison avec un électrolyte physiologiquement acceptable et/ou une ou plusieurs autres substances à activité osmotique.

2. Procédé selon la revendication 1, caractérisé en ce que l'on utilise un ester d'amidon ayant une masse moléculaire (M̅w̅) de plus de 1000 daltons, en particulier de 100 000 à 200 000 daltons, et une substitution molaire de 0,1 à 1,5, en particulier de 0,3 à 0,5.

3. Procédé selon la revendication 1 ou 2, caractérisé en ce que l'on utilise un amidon acétylé comme ester d'amidon.

4. Procédé selon la revendication 2 ou 3, caractérisé en ce que l'on utilise comme amidon acétylé un amidon acétylé ayant une masse moléculaire (M̅w̅) de 100 000 à 200 000 daltons, et une substitution molaire de 0,1 à 0,7, en particulier de 0,3 à 0,5.

5. Procédé selon l'une des revendications 1 à 4, caractérisé en ce que l'on utilise 2 à 6% en poids de l'ester d'amidon.

6. Procédé selon la revendication 5, caractérisé en ce que, comme autres substances à activité osmotique, on utilise des polyols, des monosaccharides, des disaccharides et/ou des aminoacides.

7. Procédé selon la revendication 6, caractérisé en ce que, comme autre substance à activité osmotique, on utilise le glucose.
